(19) Europäisches Patentamt
European Patent Office
Office européen des brevets

(11) Publication number: 0 195 397
B1

(12) **EUROPEAN PATENT SPECIFICATION**

(45) Date of publication of patent specification: 13.12.89

(51) Int. Cl.[4]: A 23 K 1/16, A 61 K 31/275

(21) Application number: 86103581.4

(22) Date of filing: 17.03.86

# (54) m-Cyanophenethanolamines as animal growth promoters and antilipogenic agents.

(30) Priority: 21.03.85 US 714239

(43) Date of publication of application:
24.09.86 Bulletin 86/39

(45) Publication of the grant of the patent:
13.12.89 Bulletin 89/50

(84) Designated Contracting States:
AT BE CH DE FR GB IT LI LU NL SE

(56) References cited:
EP-A-0 103 830
GB-A-1 154 193
US-A-4 404 222
CHEMICAL ABSTRACTS, vol. 67, no. 17, 23rd October 1967, page 7607, no. 80849e, Columbus, Ohio, US; E. GRANA et al.: "The determination of the blood lactic acid as a test for evaluating bêta-adrenergic compounds", & FARMACO, ED. SCI. 22(8), 573-81, 1967

**The file contains technical information submitted after the application was filed and not included in this specification**

(73) Proprietor: AMERICAN CYANAMID COMPANY
1937 West Main Street P.O. Box 60
Stamford Connecticut 06904-0060 (US)

(72) Inventor: Asato, Goro
10 Maddock Road
Titusville New Jersey 08560 (US)

(74) Representative: Wächtershäuser, Günter, Dr.
Tal 29
D-8000 München 2 (DE)

(56) References cited:
FARMACO EDIZIONE SCIENTIFICA, vol. 29, no. 2, 1974, pages 159-166, Pavia, IT; L. VILLA et al.: "Stereochimica di 1-aril-2-isopropilaminopropan-1-oli"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 195 397 B1

Courier Press, Leamington Spa, England.

**Description**

Background of the Invention

The *m*-cyanophenethanolamine compounds such as *m*-[1-hydroxy-2-(isopropylamino)ethyl]benzonitrile and *m*-[2-(*tert*-butylamino)-1-hydroxethyl]benzonitrile are described by L. Villa, Coll., Il Farmaca-Ediz. Sci., *29*, 162—166. However, these compounds are not known to be effective for increasing the growth rate of animals and/or inhibiting the deposition of body fat in said animals or reducing the body fat thereof.

Although J. A. Kiernan and P. K. Baker, United States Patents 4,404,222 and 4,407,819, issued September 13, 1983 and October 4, 1983, respectively, have disclosed the use of certain phenethanolamines for increasing the growth rate and improving the lean meat to fat ratio of meat-producing animals, they have neither indicated nor suggested that the compounds useful in the present invention would be useful for increasing the growth rate of animals or reducing the body fat thereof.

Recently, European Patent Application 103830 was published describing substituted phenylethylamine derivatives which were said to be growth promoters for pigs, cows, poultry, cats, dogs, rabbits, fur animals, fish, and reptiles.

GB—A—1 154 193 describes the result of studies of the biological activity of 2,3-substituted phenyl ethanol-N-isopropylamines. It is indicated in this document that some of the compounds studied have a low β-mimetic and β-lytic activity.

Summary of the Invention

This invention relates to a method for increasing the growth rate of warm-blooded animals, such as meat-producing animals and domestic pets by orally or parenterally administering thereto a growth rate enhancing amount of a *m*-cyanophenethanolamine compound, namely *m*-[2-(*tert*-butylamino)-1-hydroxyethyl]benzonitrile. It also relates to a method for reducing the body fat of said animals and/or inhibiting the deposition of body fat in such animals, by orally or parenterally administering to said animals a body fat inhibiting or reducing amount of the *m*-cyanophenethanolamine compound.

The invention is especially useful to poultrymen, farmers and ranchers, in the rearing of poultry, cattle, swine, sheep and goats, since it provides them with an effective and convenient means for obtaining larger leaner animals having improved carcass quality and increasing weight. This invention is likewise useful to pet owners and veterinarians, since it provides them with an effective and convenient means for reducing the body fat of pet animals, thereby yielding leaner, more vibrant pets.

The compounds useful in the method of the present invention exhibit surprisingly low $\beta_1$ heart stimulant activity. As such, they have a significantly improved or added margin of safety in their use over art compounds that exhibit substantial $\beta_1$ heart stimulant activity.

Description of the Preferred Embodiments

The above identified compounds which are useful in the present invention may be illustrated by formula I:

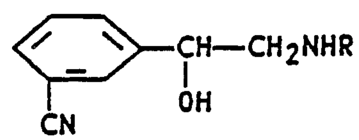

(I)

wherein R is *tert*-butyl; and the optical isomers thereof and the non-toxic, pharmacologically acceptable acid addition salts thereof.

In accordance with this invention a formula I compound can be administered either orally or parenterally to domestic or farm animals with resultant increases in growth rates and/or reduction of body fat or inhibition of fat deposition. The active compounds may be mixed directly with animal feeds or, preferably, prepared in the form of an animal-feed premix, animal-feed concentrate, or feed supplement which can be blended with the feed or applied as a top dressing thereto. Regardless of which procedure is selected for administration, the active compound should be proffered in an amount sufficient to provide from about 0.05 to 200 ppm of active compound of preferably 0.05 to 100 ppm of active compond in the total feed.

Animal-feed premixes, supplements or concentrates are readily prepared by mixing, on a weight basis, about 0.5 to 50% of a benzonitrile derivative of a pharmacologically acceptable salt thereof with about 50% to 99.5% of a edible diluent. Diluents suitable for use in the manufacture of animal-feed supplements, concentrates, and premixes include: corn meal, soybean meal, bone meal, alfalfa meal, cottonseed oil meal, urea, molasses, and other similar materials. Use of the diluents in feed supplements, concentrates, and premixes improves uniformity of distribution of the active ingredient in the finished feed.

Feeds for swine, cattle, sheep, and goats generally contain about 0.05 to 200 grams of active ingredient per ton of feed with an optimum level of about 0.125 to 100 grams of active ingredient per ton of feed. Poultry and domestic-pet feeds are usually prepared in such a manner as to contain from about 0.05 to 100 grams and most preferably about 0.1 to 100 grams of active ingredient per ton of feed.

For parenteral administration of the active ingredient, the formula I benzonitrile or pharmacologically acceptable salt thereof, is formulated as a paste or pellet and administered to the animals by subcutaneous

injection. This procedure involves injection of a sufficient amount of the formulated paste or a sufficient number of pellets which contain the formula I compound to provide the animals with about 0.01 to 100 mg/kg of body weight/day of said active compound. The preferred dosage for swine, cattle, sheep, and goats ranges from about 0.01 to 50 mg/day/kg of body weight of the benzonitrile or pharmacologically acceptable salt thereof. The preferred dosae of the formula I compound for poultry and domestic pets ranges from about 0.001 to 10 mg/day/kg of animal body weight.

Paste or gel formulations suitable for subcutaneous injection can be prepared by dispersing a formula I benzonitrile or pharmacologically acceptable salt thereof in a pharmacologically acceptable diluent, such as butylene glycol, peanut oil, corn oil, sesame oil, or a clear aqueous, thermally reversible, gel composition.

A typical gel formulation can be prepared in accordance with the following procedure.

The gellant phase is prepared by slurring the gellant 15% to 50% and preferably 15% to 35% by weight of formulation in propylene glycol 14% to 30% by weight for 15 minutes to one hour under reduced pressure 25 to 50 mm Hg at room temperature. The gellant selected is a nonionic surfactant of structure α-hydro-Ω-hydroxy-poly-(oxyethylene)poly(oxypropylene)poly(oxyethylene)block copolymer, average molecular weight 12,500; mp 56°C; Brookfield viscosity of 3,100 at 77°C; surface tension of a 0.1% aqueous solution: 40.6 dynes/cm (measured with a duNouy tensiometer;.

As aqueous solution containing the remaining ingredients may be prepared by dissolving or dispersing the benzonitrile or an acceptable salt thereof, preferably the hydrochloride, in amounts of from about 3% by weight to about 25% by weight and preferably 6% to 12% by weight of final formulation in deionized or distilled water used in amounts of from about 15% by weight to about 50% by weight and preferably 35% to 45% by weight of formulation. This solution is buffered by dissolving 1.5% by weight of citric acid and 1.0% by weight of trisodium citrate to provide a pH range at which long-term chemical stability of the components is achieved, i.e., pH 3—3.5.

Optional components, which may be incorporated into the above solution at this stage are:

a. Benzyl alcohol added in amounts of from about 0.5% by weight to about 1.5% by weight and preferably 1.5% by weight of formulation as an antimicrobial preservative;

b. The yellow dye C.I. Acid yellow No 23, ("tartrazine," F. D. & C. yellow No 5; 4,5-dihydro-5-oxo-1-(4-sulfophenyl)-4-[(sulfophenyl)azo]-1H-pyrazole-3-carboxylic acid trisodium salt) used as a coloring agent in amounts of from about 0.01% by weight to about 0.03% by weight and preferably 0.01% by weight of formulation;

c. An antifoaming agent comprising a mixture of dimethylpolysiloxanes of structure:

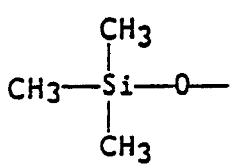 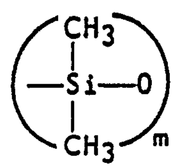 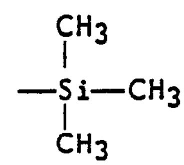

and silica gel, wherein the calculated average value of m is 200 to 350, the mixture is a water-white viscous oil-like liquid; d = 0.965—0.970; $n_D^{25}$ about 1.404; viscosity about 60,000 centistokes used in amount of from 0.001 to 0.02% by weight and preferably 0.02% by weight of formulation.

The lipolytic and antilipogenic gel of this invention is prepared by simply mixing either of the above gellant phases and the aqueous solution from one-half hour to two hours under reduced pressure of from 10 to 100 mm Hg and preferably 25 to 50 mm Hg at ambient temperatures of from 20°C to 60°C, without the requirements of either additional heating or cooling. This procedure gives an air-free gel which is suitable for administering exact dosages of the antilipogenic composition by volume.

Pellets for subcutaneous injection can be prepared by mixing a formula I, benzonitrile or a pharmacologically acceptable salt thereof with a suitable diluent, such as montan wax, carbowax, carnauba wax or the like, and compressing the same into a pellet form. A lubricant such as magnesium or calcium stearate can be added to improve the pelleting process if so desired.

In order to obtain the drug dosage levels necessary to achieve desired results (i.e. increase in growth rates and/or improvement in lean meat to fat ratios), it may be necessary to administer multiple pellets. Also, implants may be made periodically during treatment periods in order to maintain proper drug levels in the animals.

In addition to improve growth rates and reduce body fat obtained with the compounds of this invention, administration of formula I compounds to meat-producing animals frequently results in enhanced efficiency of feed utilization thereby and reduced feed costs to bring the animals to market weight. With the use of materials and methods revealed in the present invention, producers can market superior quality meat animals in a short period of time while incurring minimum feed costs.

The following non-limiting examples further serve to illustrate the invention.

Example 1

Preparation of *m*-[2-(*tert*-butylamino)-1-hydroxyethyl]benzonitrile

To a mixture of 250 mL p-dioxane, 15 mL of water and 15.4 g selenium dioxide maintained at 60°C is added (10 g, 0.069 mol) of *m*-acetylbenzonitrile. The mixture is heated to refluxing temperature for 20 hours then filtered, stripped, partitioned between dichloromethane and water (500 mL/3 × 200mL), then dried with dichloromethane and stripped to 200 mL. To the remaining mixture is added *tert*-butylamine in two 10.1 g portions. The mixture is stirred for one hour at room temperature, stripped at 35°C over a one hour period, then triturated with anhydrous ethylene oxide and filtered, leaving a white solid. The white solid is added to 50 mL of ethanol and the mixture treated with four 10.5 g samples of sodium borohydride. The resulting mixture is stirred for five hours, then stripped, and the remaining residue partitioned between water and dichloromethane (3 × 200 mL/500 mL). After partitioning the dichloromethane portion is dried with magnesium sulfate and stripped to give a white solid. After drying over refluxing acetone the product, referred to above, is obtained and has a melting point of 86°C to 87°C. Product analysis showed the following:

% calc'd. C—71.56; H—8.26; N—12.84

% Found C—71.83; H—8.16; N—12.72

The reaction described above may be illustrated as follows:

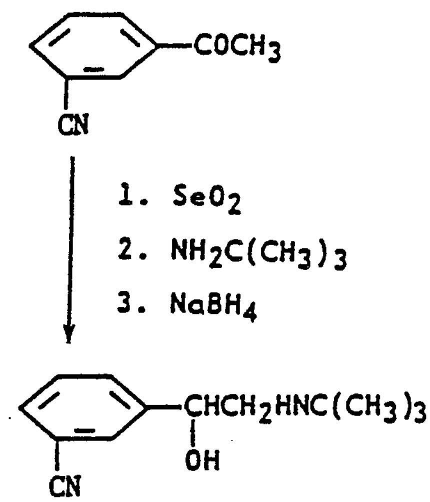


Example 2

Evaluation of test compounds as animal growth promoters and antilipogenic agents-mouse tests

CFI female mice from Carworth Farms are received when they are six-weeks old. They are housed ten to a cage in air-conditioned rooms (22°C to 25°C) with automatically-controlled lights, 14 hours on and ten hours off. The basal diet used in these studies is Purina Laboratory Chow (see description below) which is supplied ad libitum.

The following is a description of the diet to which the growth-promoting compounds were added.

DIET

Guaranteed Analysis

| | |
|---|---|
| Crude Protein not less than | 23.0% |
| Crude fat not less than | 4.5% |
| Crude fiber not more than | 6.0% |
| Ash not more than | 9.0% |

Ingredients

Meat and bone meal, dried skimmed milk, wheat germ meal, fish meal, animal liver meal, dried beet pulp, ground extruded corn, ground oat groats, soybean meal, dehydrated alfalfa meal, cane molasses, animal fat preserved with BHA, vitamin $B_{12}$ supplement, calcium pantothenate, chlorine chloride, folic acid, riboflavin supplement, brewer's dried yeast, thiamin, niacin, vitamin A supplement, D-activated plant sterol, vitamin E supplement, calcium carbonate, dicalcium phosphate, iodized salt, ferric ammonium citrate, iron oxide, manganous oxide, cobalt carbonate, copper oxide, zinc oxide. Water is also allowed ad libitum.

Thirteen days after arrival, the mice are weighed in groups of ten and assigned at random to the different treatments. Each of the treatments is tested in three replicates, i.e., in three cages of ten mice each. There are ten cages of ten control mice each. Drugs are mixed in the diet at the dosage level indicated. Feed and water are offered ad libitum for a 12-day test period. Feed spilled is collected during the test period. At the end of the test perod, the collected feed is weighed, and the mean feed consumption per cage of ten mice is degermined for each treatment. The mice are weighed as a group of ten, and the weight gain determined. The mice are sacrificed by cervical dislocation. The right uterine fat pad of each mouse is removed. The fat pads for each cage of ten mice are weighed as a unit. Reduction in fat pad weights of animals is generally indicative of a reduction of total body fat of the treated animals.

Moreover, when a significant decrease in body fat is coupled with a marked improvement in weight gain in the treated animals, we have found that the lean meat to fat ratio of said-treated animals is substantially improved.

Data obtained are reported in Table I below.

TABLE I

Evaluation of test compounds as animal growth promoters and antilipogenic agents — mouse test

| Structure | Dosage (ppm) | Gain (grams) | % Increase in body weight vs controls | % Reduction in fat pad weight vs controls |
|---|---|---|---|---|
| 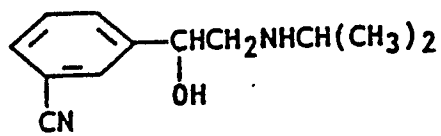 (comparative) | 200 | 39.0 | +36.8 | −11.7 |
| | 100 | 37.3 | +30.9 | −9.1 |
| | 50 | 37.4 | +31.2 | −21.2 |
| | 25 | 37.1 | +30.2 | +0.8 |
| | 12 | 29.4 | +3.2 | +0.5 |
| | 6 | 23.4 | −17.9 | −13.7 |
| | 3 | 32.4 | +13.7 | −8.7 |
| 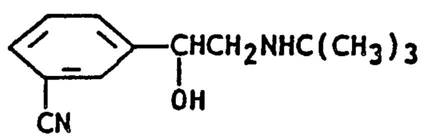 | 200 | 33.2 | +16.5 | −19.5 |
| | 100 | 36.1 | +26.7 | −16.9 |
| | 50 | 41.1 | +44.2 | −11.6 |
| | 25 | 35.9 | +26.0 | +5.2 |
| | 12 | 30.5 | −7.0 | +12.1 |
| | 6 | 37.4 | +54.4 | −8.3 |
| | 3 | 29.3 | +2.8 | −8.3 |

Example 3

Determination of beta-1 adrenergic activity of experimental repartitioning compounds

Beta-1 adrenergic activity of experimental compounds is identified by radioactive ligand-binding studies by using beta adrenergic receptor membranes and adrenergic antagonist (H3) dihydroalprenolol (DHA). Binding of (H3) DHA to membrane fractions is assayed by filtration on glass fiber filters. Specific binding is defined as total radioactivity bound minus nonspecific bound radioactivity, i.e., binding in the presence of 100 µM non-radioactive DHA. Results are expressed as the concentrations of the compound required to displace 50% radioactive ligand from binding of beta adrenergic receptors (K—50).

In these tests, beta adrenergic receptor membranes are incubated with radioactive dihydroalprenolol (H3 DHA) and experimental compounds. The bound radio-ligand is separated by filtration on glass fiber filters and counted by scintillation counter.

Radioactive ligand, H3 dihydroalprenolol (DHA) and aquasol are purchased from New England Nuclear Corporation, DMSO is supplied by J. T. Baker Company, and all other chemicals are obtained from Sigma Chemical Company.

Beta-1 adrenergic receptor membranes are isolated from turkey and rat erythrocytes respectively using the procedures of:

Hancock, A., DeLean, A., Lefkowitz, R. J. Mol. Pharmacol. 16:1, 1979;

DeLean, A, Hancock A., Lefkowitz, R. J. Mol. Pharmacol. 21:5, 1979; or

Lefkowitz, R. J., Stadel, J. M., and Caron, M. G. Ann. Rev. Biochem 52:157, 1983.

Freshly drawn heparinized whole blood from turkeys and rats is centrifuged for five minutes (4 × 500 g). The plasma is withdrawn; the remaining erythrocytes are suspended in 150 mM sodium chloride solutions and centrifuged. The cells are resuspended and centrifuged twice more. The cells are hemolyzed in 10 volume of cold distilled water containing 2 mM dithiothreitol, 100 µM phenylmethyl sulfonyl fluoride, 5 µg/mL leupeptin, 200 µg/mL bacitacin, 0.1% bovine serum albumin, and 10 units/mL aprotonin, and centrifuged at 3000 Xg for five minutes. The bottom gelatin layer is discarded. The top layer is suspended and centrifuged five times at 3000 Xg and resuspended in tris buffer (145 mM NaCl, 1mM ethylene glycol-bis (β-aminoethyl ether) N,N,N,N, tetraacetic acid, 2 mM $MgCl_2$, 10 mM tris, and 10% glycerol, pH 7.4). The resuspended membranes are further purified with differential centrifugation by using sucrose albumin cushion, and subsequently stored in small aliquots at −70°C. The protein concentration of receptor membranes is determined by the biuret method.

To duplicate incubation, tubes are added 200 µl suspended membranes (200 µg protein), 24 µl of compound in solution or buffer and 25 µl (H3) dihydroalprenolol (2000,000 CPM). Tubes were incubated at room temperature (23°C) for an hour and the filtered rapidly under vacuum through Whatman GF/B filters. The filters are rinsed three times with 5 mL of cold buffer (145 mM NaCl, 1 mM ethylenediaminetetraacetic acid, and 10 mM tris, pH 7.4) and subsequently counted by liquid scintillation counter in 10 mL of aquasol. Specific binding of H3 DHA is defined as the excess over blank containing 100 µM nonradioactive DHA. Six serial dilutions of each compound are tested.

Beta adrenergic activity is inversely related to K—50 (the concentrations of the compound required to displace 50% radioactive ligand from binding sites of beta adrenergic receptors, i.e., 50% binding) value.

Data obtained are reported in Table II below.

TABLE II

Beta-1 adrenergic activity determined as K—50 (the concentrations of the compound required to displace 50% radioactive ligand from binding sites of beta adrenergic receptors, i.e., 50% binding) value for benzonitrile derivatives with clenbuterol as a standard

| Compound | $\beta_1$ K—50 (μM) |
|---|---|
| 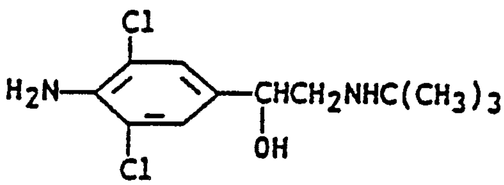 * Clenbuterol (Standard) | 0.977 |
| 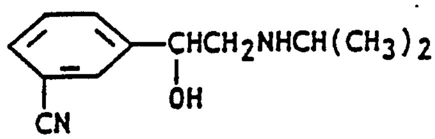 (comparative) | >100 |
| 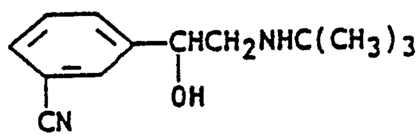 | >100 |

**Claims**

1. A method for increasing the growth rate and/or for reducing the body fat of warm-blooded animals comprising: orally or parenterally administering to said animals a growth promoting and/or at reducing amount of a compound having the structure:

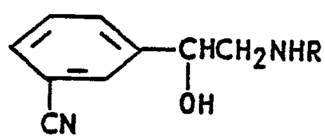

wherein R is *tert*-butyl; or an optical isomer thereof or a non-toxic pharmacologically acceptable acid addition salt of the above-said compound.

2. A method according to Claim 1, for increasing the growth rate and/or for reducing the body fat of meat-producing animals and domestic pets by orally administering thereto an edible animal feed containing from 0.05 to 200 grams of said compound per ton of edible animal feedstuff.

3. A method according to Claim 1, for increasing the growth rate and/or for reducing the body fat of meat-producing animals and domestic pets by parenterally administering thereto by subcutaneous injection of an implant composition containing sufficient active compound to provide said animals with from 0.001 to 100 mg/kg/day of body weight of said compound.

4. A method according to Claim 1, wherein said warm blooded animals are meat-producing animals selected from the group consisting of poultry, sheep, cattle, swine and goats or domestic pets selected from the group consisting of dogs and cats.

5. A method according to Claim 2, wherein the animals are poultry, cattle, sheep, swine or goats.

6. A method according to Claim 1, wherein the compound is *m*-[2-(*tert*-butylamino)-1-hydroxyethyl]-benzonitrile.

7. An animal feed composition comprising an edible animal feed containing from 0.05 to 200 grams per ton of feed of a compound of the formula:

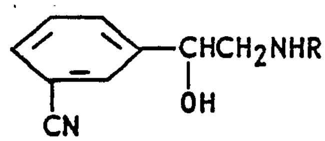

wherein R is *tert*-butyl; or an optical isomer thereof or a non-toxic pharmacologically acceptable acid addition salt of the above-said compound.

**Patentansprüche**

1. Verfahren zur Steigerung der Wachstumsrate und/oder zur Reduzierung des Körperfetts von warmblütigen Tieren, umfassend die orale oder parenterale Verabreichung einer wachstumsfördernden und/oder fettreduzierenden Menge einer Verbindung der folgenden Struktur an die Tiere

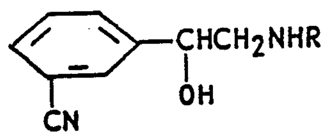

wobei R für tert-Butyl steht; oder ein optisches Isomeres derselben oder ein nicht toxisches, pharmakologisch akzeptables Säureadditionssalz der oben genannten Verbindung.

2. Verfahren gemäß Anspruch 1 zur Steigerung der Wachstumsrate und/oder zur Reduzierung des Körperfetts von fleischerzeugenden Tieren und Haustieren durch orale Verabreichung eines eßbaren Tierfutters, enthaltend von 0,05 bis 200 g der erwähnten Verbindung/Tonne des eßbaren Tierfutters.

3. Verfahren gemäß Anspruch 1 zur Steigerung der Wachstumsrate und/oder Reduzierung des Körperfetts von fleischerzeugenden Tieren und Haustieren durch parenterale Verabreichung mittels subkutaner Injektion einer Implantatzusammensetzung, enthaltend ausreichend Wirkstoff, um die Tiere mit von 0,01 bis 100 mg der Verbindung/kg Körpergewicht/Tag zu versorgen.

4. Verfahren gemäß Anspruch 1, wobei die warmblütigen Tiere fleischerzeugende Tiere sind, ausgewählt aus der Gruppe bestehend aus Geflügel, Schafen, Rindern, Schweinen und Ziegen, oder Haustiere, ausgewählt aus der Gruppe bestehend aus Hunden und Katzen.

5. Verfahren gemäß Anspruch 2, wobei die Tiere Geflügel, Rinder, Schafe, Schweine oder Ziegen sind.

6. Verfahren gemäß Anspruch 1, wobei die Verbindung m-[2-(tert-Butylamino)-1-hydroxyethyl]benzonitril ist.

7. Eine Tierfutterzusammensetzung, umfassend ein eßbares Tierfutter enthaltend pro Tonne Futter 0,05 bis 200 g einer Verbindung der Formel

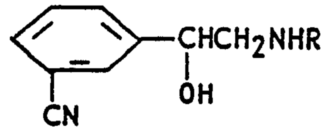

wobei R für tert-Butyl steht; oder ein optisches Isomeres derselben oder ein nicht toxisches, pharmakologisch akzeptables Säureadditionssalz der oben genannten Verbindung.

**Revendications**

1. Procédé pour l'augmentation du taux de croissance et/ou pour la diminution de la graisse corporelle d'animaux à sant chaud, comprenant l'administration par voie orale ou parentérale auxdits animaux d'une quantité stimulante la croissance et/ou diminuant les matières grasses, d'un composé de structure

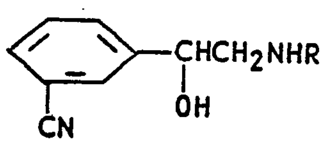

dans laquelle R est le radical tert-butyle, ou d'un isomère optique de celui-ci ou d'un sel d'addition avec un acide pharmacologiquement acceptable, non toxique, dudit composè ci-dessus.

2. Procédé selon la revendication 1 pour l'augmentation du taux de croissance et/ou la diminution de la graisse corporelle d'animaux producteurs de viande et d'animaux domestiques familiers, par

administration par voie orale à ceux-ci d'un aliment comestible pour animaux, contenant de 0,05 à 200 g dudit composé par tonne d'aliment comestible pour animaux.

3. Procédé selon la revendication 1 pour l'augmentation du taux de croissance et/out la diminution de la graisse corporelle d'animaux producteurs de viande et d'animaux domestiques familiers, par administration par voie parentérale à ceux-ci, par injection sous cutanée, d'une composition d'implant contenant une quantité suffisante de composé actif pour fournir ausdits animaux de 0,01 à 100 mg dudit composé par kg de poids corporel et par jour.

4. Procédé selon la revendication 1, dans lequel lesdits animaux à sang chaud sont des animaux producteurs de viande choisis parmi les volailles, ovins, bovins, porcins et chèvres, ou des animaux domestiques familiers choisi parmi le chien et le chat.

5. Procédé selon la revendication 2, dans lequel les animaux sont des volailles, bovins, ovins, porcins ou chèvres.

6. Procédé selon la revendication 1, dans lquel le composé est le m-[2-(tert-butylamino)-1-hydroxy-éthyl]benzonitrile.

7. Composition d'aliment pour animaux comprenant un aliment comestible pour animaux contenant, par tonne d'aliment, de 0,05 à 200 d'un composé de formule

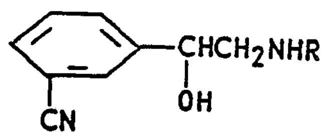

dans laquelle R est le radical tert-butyle, ou d'un isomère optique de celui-ci ou d'un sel d'addition avec un acide pharmacologiquement acceptable, non toxique, dudit composé ci-dessus.